# EUROPEAN PATENT APPLICATION

(11) **EP 2 133 072 A1**
(43) Date of publication of application: **16.12.2009**
(21) Application number: 08010804.6
(22) Date of filing: 13.06.2008
(51) Int. Cl.: A61K 9/50, C07D 333/20, A61K 9/26, A61K 9/30

(54) **Gastro-resistant pharmaceutical oral compositions comprising duloxetine or its pharmaceutically acceptable derivatives**

(71) Applicant: KRKA, D.D., Novo Mesto, 8501 Novo mesto (SI)
(72) Inventor: Zajc, Natalija, 8000 Novo mesto (SI); Vrecer, Franc, 8351 Straza pri Novem mestu (SI); Benkic, Primoz, 1000 Ljubljana (SI); Bukovec, Polona, 8000 Novo mesto (SI); Tihi, Jaroslav, 8000 Novo mesto (SI)
(74) Representative: HOFFMANN EITLE

(57) **Abstract**

The present invention relates to a pharmaceutical composition comprising an active core comprising duloxetine or its pharmaceutically acceptable derivatives, a separating layer comprising a water soluble alkaline substance and a gastro-resistant coating comprising a gastro-resistant polymer selected from methacrylic acid copolymers and optionally an over-coating layer.

## Description

### FIELD OF THE INVENTION

The present invention belongs to the field of pharmaceutical technology and provides a pharmaceutical composition comprising duloxetine or its pharmaceutically acceptable derivatives and methods of manufacture thereof. More particularly, the present invention encompasses the pharmaceutical composition comprising an active core comprising duloxetine or its pharmaceutically acceptable derivatives, a separating layer comprising water soluble alkaline substance and a gastro-resistant coating comprising gastro-resistant polymer selected from methacrylic acid copolymers and optionally an over-coating layer.

### BACKGROUND OF THE INVENTION

Duloxetine, (*S*)-(+)-*N*-methyl-3-(1-naphthalenyloxy)-3-(2-thienyl)propanamine, with a chemical structure of formula I 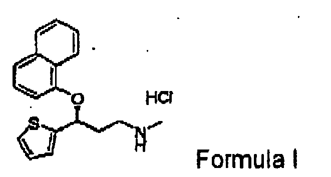 is a dual serotonin and norepinephrine re-uptake inhibitor. Duloxetine has particular therapeutic utility as an anti-depressant. It may be prepared in the form of salts, such as for example oxalate salt, maleate salt, hydrochloride salt, di-p-toluyl tartarate salt, fumaric salt, citric salt, mandelic salt, or any other pharmaceutically suitable salt and in the form of different solvates in any enantiomeric form.

Duloxetine and its pharmaceutically acceptable salts were first described in EP 273658. Duloxetine hydrochloride is currently marketed by Eli Lilly under the trade name CYMBALTA^{®}.

Duloxetine in the form of pharmaceutically acceptable salts is known to be unstable in acidic environment. In order to prevent its acid induced degradation, specially to 1-naphthol which is known to be toxic for humans, duloxetine is formulated into coated pellets where at least one coating is insoluble at pH lower than 5.5. Insolubility of coating in acidic media can be ensured by using polymers which are not soluble in acidic media, but are readily soluble at the pH of small intestine, i.e. a pH higher than 5.0. In addition, it is known that duloxetine readily reacts with polymer degradation products or residual free acids present in the polymer enteric coating. Hence, at least one separating or intermediate layer should be applied between duloxetine and enteric coating polymer. The separating layer physically keeps the components in the core and enteric layer from coming into direct contact with each other. Moreover, a separating layer has a smoothing function which means that it improves the coverage of the enteric layer and avoids thin spots in it. It can also act as a diffusional barrier during storage and it can be used as a light barrier as well.

Different approaches of formulating duloxetine or its pharmaceutically acceptable salts in solid dosage forms were used. Mainly the disclosed pharmaceutical compositions comprise an inert core with active layer, one or more separating layer, gastro-resistant layer and optional finishing layer.

EP 693282 A describes enteric pellets comprising a core consisting of duloxetine and pharmaceutically acceptable excipient, an optional separating layer and an enteric layer comprising hydroxypropylmethylcellulose acetate succinate and an optional finishing layer. The separating layer is composed of coherent or polymeric materials and finely powdered solid excipients which constitute fillers.

US 2007/0004795 A1 discloses a palatable oral pharmaceutical composition comprising comprising duloxetine or its pharmaceutically equivalent derivatives in a formulation comprising at least one buffering agent.

US 2006/0165776 A1 refers to duloxetine pharmaceutical composition comprising a core with duloxetine, an intermediate and an enteric layer, wherein the intermediate layer includes organic or inorganic polymers, sugars and like.

In WO 2007034503 a controlled release dosage form of duloxetine for once daily administration, which comprises duloxetine or its pharmaceutically acceptable salts, pharmaceutically acceptable polymeric carrier and solubility enhancer is described.

EP 1820498 discloses an enteric-coated preparation, wherein the enteric coating material is selected from the group consisting of hydroxypropylmethyl cellulose acetate succinate, hydroxypropylmethyl cellulose phthalate, methacrylic acid copolymer and carboxymethylethyl cellulose.

WO 2007093439 discloses the pharmaceutical composition with duloxetine or its pharmaceutically acceptable salts comprising a duloxetine-layer applied on an inert bead, a separating layer providing smoothing and physical barrier function, inhibition of interaction between core and enteric layer and diffusional barrier and one or more enteric coating layers.

A multiple unit composition comprising enteric coated pellets is described in WO 2007122478 wherein each pellet comprises a core with active ingredient, optionally a separating layer and at least two enteric layers. The separating layer comprises binder, that is dispersed or dissolved in a solvent and optionally other pharmaceutically acceptable ingredients.

WO 2007139886 discloses a duloxetine hydrochloride delayed release formulation comprising an inert core, a drug layer, a separating layer and an enteric layer comprising at least one of a methacrylic acid copolymer and hydroxypropyl methyl cellulose ophthalate and optionally a finishing layer. The separating layer preferably comprises a coating agent and optionally one or more additional pharmaceutically acceptable excipients. According to the description the separating layer performs the function of providing a smooth base for the application of the enteric layer, prolonging the formulation's resistance to the acidic environment of the stomach, improving stability of the formulation by inhibiting interaction between duloxetine hydrochloride and the enteric layer and improving stability of the formulation by protecting the duloxetine hydrochloride from exposure to light.

WO 2008006506 discloses a pharmaceutical preparation for oral administration with controlled active ingredient release in the small intestine comprising an inner layer for controlling the active ingredient release and a covering layer that is resistant to gastric juice wherein the inner layer is constructed from at least two diffusion layers whose permeability for the diffusing active ingredient decreases from the inside to the outside.

Delayed release composition of duloxetine or its pharmaceutically acceptable acid additional salts that may comprise a separating layer is disclosed in WO 2008020286. The separating layer provides stability by inhibiting direct contact of duloxetine and the enteric coating polymer and protection to the core during its passage from the stomach to the intestines. It is said that the separating coat does not affect the dissolution of the composition.

WO 2008019712 discloses a pharmaceutical preparation comprising a core with an active ingredient and with an organic acid and/or with the salt of an organic acid and a coating comprising methacrylate copolymer having not more than 15% by weight of cationic or anionic groups.

As shown above many different approaches how to avoid degradation of an active substance that is unstable in acidic media, such as for example duloxetine or its pharmaceutically acceptable derivatives, have already been disclosed in the prior art. However, none of the prior art references refers to the problem of physical interaction between duloxetine or its pharmaceutically acceptable derivatives in the core and gastro-resistant polymer, particularly methacrylic acid copolymers, in the gastro-resistant coating that could occur during in vitro or in vivo dissolution of duloxetine. The use of methacrylic acid coplymers in gastro-resistant coating of the pharmaceutical composition comprising duloxetine or its pharmaceutically acceptable derivatives is very attractive as the manufacturing process of coating using this type of polymers is simpler because no cooling of the coating dispersion is needed as in case of the usage of other gastro-resistant polymers as for example when hydropropylmethyl cellulose acetatesuccinate is used. Similarly, there is no need for the use of organic solvents as in the case of cellulose acetatephtalate. Suprisingly low and incomplete dissolution of duloxetine or its pharmaceutically acceptable derivatives was found in cases when the separating layer is composed of water soluble polymer and water soluble additives and outer coating based on methacrylic acid copolymer such as Eudragit L type obtainable from Evonic Roehm GmbH, which is unsoluble in pH below 5.5.

Therefore, it would be a significant contribution to the art to provide a chemically and physically stable pharmaceutical composition comprising said active substance while said pharmaceutical composition is prepared in an efficient and economical way. The above mentioned problems have been solved by preparing a pharmaceutical composition comprising an active core comprising duloxetine or its pharmaceutically acceptable derivatives, a separating layer comprising one or more water soluble alkaline substances wherein the coating dispersion with added water soluble alkaline substance preferably have a pH in the range from about 6 to about 10 and a gastro-resistant coating comprising gastro-resistant polymer selected from methacrylic acid copolymers. An advantage of preparing said formulation is an increased dissolution rate with regard to the pharmaceutical composition without water soluble alkaline substance in the separating layer.

### SUMMARY OF THE INVENTION

The first embodiment of the present invention relates to the pharmaceutical composition, which comprises an active core comprising duloxetine or its pharmaceutically acceptable derivatives, a separating layer comprising water soluble alkaline substance and a gastro-resistant coating comprising gastro-resistant polymer selected from methacrylic acid copolymers and optionally an over-coating layer.

The second embodiment of the present invention relates to the preparation of the pharmaceutical composition which comprises an active core comprising duloxetine or its pharmaceutically acceptable derivatives, a separating layer comprising water soluble alkaline substance, a gastro-resistant coating comprising gastro-resistant polymer selected from methacrylic acid copolymers and optionally an over-coating layer.

The third embodiment of the present invention relates to the use of a water soluble alkaline substance in a separating layer in order to improve dissolution of duloxetine or its pharmaceutically acceptable derivatives in pharmaceutical compositions comprising a gastro-resistant polymer, which is insoluble in aqueous solutions with pH below 4.5 and is formed onto the pellets coated with separating layer as a separate coating, particularly methacrylic acid copolymers.

The fourth embodiment of the present invention relates to the process for the preparation of duloxetine hydrochloride with appropriate chemical and enantiomeric purity and with particle size appropriate for direct use in the pharmaceutical composition according to the present invention.

### FIGURES

Figure 1: Duloxetine hydrochloride particles in a large needle-like form.
Figure 2: Particles of duloxetine hydrochloride obtained by example 7.
Figure 3: Particle size distribution of particles of duloxetine hydrochloride obtained by example 7
Figure 4: Particles of duloxetine hydrochloride obtained by example 9
Figure 5: Particle size distribution of particles of duloxetine hydrochloride obtained by example 8
Figure 6: Particles of duloxetine hydrochloride obtained by example 9 A
Figure 7: Particle size distribution of duloxetine hydrochloride obtained by example 9 A
Figure 8: Particles of duloxetine hydrochloride obtained by example 9 B
Figure 9: X-ray powder diffraction pattern of duloxetine hydrochloride prepared by the example 9 B

### DETAILED DESCRIPTION OF THE INVENTION

The first embodiment of the present invention relates to the pharmaceutical composition which comprises an active core comprising duloxetine or its pharmaceutically acceptable derivatives, a separating layer comprising water soluble alkaline substance and a gastro-resistant coating comprising gastro-resistant polymer selected from methacrylic acid copolymers and optionally an over-coating layer.

The term active core as defined herein relates to anything below the separating layer and comprising duloxetine or its pharmaceutically acceptable derivatives. The term separating layer as defined herein relates to one or more separating layers that are formed between the active core and the gastro-resistant coating and comprising water soluble alkaline substance. The term gastro-resistant coating as defined herein relates to one or more gastro-resistant coating that is formed onto the separating layer and comprising at least one gastro-resistant polymer selected from methacrylic acid copolymers. The term over-coating layer relates to a layer that is optionally formed on the gastro-resistant coating.

Unless stated otherwise, all percentages given herein is by weight. Due to nature of the preparation process of the gastro-resistant duloxetine formulations the dissolution values underlie certain variations (cf. Ph.Eur.). Values are to be understood as falling within the indicated range(s).

The term duloxetine or its pharmaceutically acceptable derivatives means duloxetine as a free base or duloxetine in the form of pharmaceutically acceptable salts and their solvates or hydrates and any mixtures thereof. The present invention contemplates use of all existing polymorphs, hydrates, solvates with pharmaceutically acceptable solvents and optical isomers (i.e. racemic mixture or individual enantiomers thereof and any mixture thereof) of these substances. Preferably duloxetine is in the form of duloxetine hydrochloride in any polymorphic form. Preferably duloxetine hydrochloride is in the polymorph form A.

The pharmaceutical composition according to the present invention can comprise duloxetine or its pharmaceutically acceptable derivatives in combination with at least one other active substance as for example, but not limited to, in combination with 5-HT_{1D} antagonist or in combination with alprenolol, spiperon, pindolol, penbutolol, propranolol, tertatolol.

Duloxetine or its pharmaceutically acceptable derivatives that can be used in the present invention as an active substance may be prepared by any method known from the literature such as for example EP 273658 and EP 650965.

The active core can be prepared from duloxetine or its pharmaceutically acceptable derivatives and at least one pharmaceutically acceptable excipient in a process which results in a matrix pellet or in a tablet. Alternatively, a core can be formed by applying a layer comprising duloxetine or its pharmaceutically acceptable derivatives onto an inert bead or inert tablet core.

The active core in the form of matrix pellet comprising duloxetine or its pharmaceutically acceptable derivatives can additionally comprise at least one pharmaceutically acceptable excipient selected from the group consisting of, but not limited to, stabilizers, fillers, disintegrating agents, wetting agents, binders and any mixtures thereof. In the said matrix pellet the active ingredient is preferably homogeneously distributed within the pellet body.

The active core can be also in the form of tablet where micro tablets with diameter below about 0.4 mm, preferably below about 0.3 mm are preferred. The active core in the form of tablet can further comprise at least one pharmaceutically acceptable excipient selected from the group consisting of, but not limited to, binders, fillers, disintegrants, lubricants, and glidants and any mixture thereof.

When the active core encompasses an inert bead, said bead can be prepared from any pharmaceutically acceptable excipient such as for example starch, sugar, microcrystalline cellulose, vegetable gums, waxes and the like. Preferably starch and sucrose can be used. The size of the beads may vary between about 0.1 and about 2 mm, preferably from about 0.25 to about 1.5 mm, most preferably from about 0.45 to about 1.0 mm.

The inert tablet core can be prepared from pharmaceutically acceptable excipient selected from the group consisting of, but not limited to, binders, fillers, disintegrants, lubricants, and glidants and any mixture thereof. The diameter of the inert tablet core can be below about 0.4 mm, preferably below about 0.3 mm.

The layer with duloxetine or its pharmaceutically acceptable derivatives may in addition to at least one active substance further comprise at least one pharmaceutically acceptable excipient selected from the group consisting of, but not limited to, stabilizers, fillers, disintegrating agents, wetting agents, binders or other pharmaceutically acceptable excipients and any mixtures thereof. The thickness of the active layer is from about 20 to about 150 µm, preferably from about 40 to about 120 µm, most preferably from about 60 to about 90 µm.

Onto the active core at least one separating layer is formed while at least one separating layer comprises water soluble alkaline substance and at least one pharmaceutically acceptable excipient. The separating layer separates the core material from the outer gastro-resistant coating. The pharmaceutical composition according to the present invention can comprise one or more separating layers between active core and gastro-resistant coating, wherein at least one of the separating layers comprises the water soluble alkaline substance.

The water soluble alkaline substance present in at least one separating layer is any pharmaceutically acceptable excipient, which, when dissolved in concentration range 1 to 10 w/vol% in purified water gives the pH of solution at 25°C in the range 7 to 13, preferably 8 to 12 and most preferably 8.5 to 10.5 and/or which, when incorporated into the dispersion for coating, results in a pH of the coating dispersion in a range 6-10. Water-solubility in the context of the present invention means that at least ... g of the substance can be dissolved in 100 ml of pure deionised water at 25°C. The water soluble alkaline substance can be selected from, but not limited to, sodium or potassium salt of phosphoric, carbonic, citric or other suitable weak inorganic or organic acid, or suitable organic base, including basic amino acid, their esters and salts and amino carbohydrate derivatives such as for example meglumine or any combination thereof. Preferably, sodium phosphate such as disodium hydrogen phosphate in anhydrous or hydrated form is included in the pharmaceutical composition according to the present invention as a water soluble alkaline substance. It is preferable that a water soluble alkaline substance is used, which results in a pH value of water solution above 9 under the following conditions: ....

The water soluble alkaline compound in the separating layer is present in an amount from 0.1 % to 10%, more preferably from 0.5% to 5% and most preferably from 1% to 2% by weight of the total weight of the pharmaceutical composition of the invention.

Primary function of the separating layer comprising the water soluble alkaline substance is to prevent interactions between gastro-resistant polymer and duloxetine or its pharmaceutically acceptable derivatives during in-vitro [b1] dissolution.

The water soluble alkaline substance used in the separating layer of the pharmaceutical composition according to the present invention preferably increases the pH of the coating dispersion to a pH above 5, more preferably above 7 and even more preferably above 9 when measured under the following conditions: ... [b2] Talc or other compounds may be added to increase the thickness of the layer and thereby strengthen the diffusion barrier or to prevent stickiness of the pellets during coating or drying.

An optionally present separating layer that does not comprise water soluble alkaline substance according to the present invention may comprise at least one pharmaceutically acceptable excipient selected from, but not limited to, stabilizers, fillers, disintegrating agents, wetting agents, binders or other pharmaceutically acceptable ingredients, and any mixtures thereof.

The thickness of the separating layer is from about 5 to about 80 µm, preferably from about 7.5 to about 30 µm.

One or more gastro-resistant coatings are applied onto the core covered with at least one separating layer comprising water soluble alkaline substance by using a suitable coating technique. At least one of these gastro-resistant coatings comprises at least one methacrylic acid copolymer and/or at least one further excipient selected from the group consisting of, but no limited to, plasticizers, antitacking agents, pH modifiers, pigments or colorants, surface active substances and optionally other inactive ingredients known from the state of the art such as those described for example in Practical Course in Film Coating of Pharmaceutical Dosage forms with Eudragit published in 1999 by Röhm GmbH.. To prepare the gastro-resistant coating, a solution or dispersion of one or more methacrylic acid copolymers can be used. For example, a copolymer of methacrylic acid and ethyl acrylate is suitable for this purpose. Suitable methacrylic acid copolymers and other materials for gastro-resistant coatings are disclosed for instance in EP 1820498 and and EP 1747776. Preferably methacrylic acid copolymers such as Eudragit L, Eudragit S or Eudragit FS types can be used.

The gastro-resistant coating comprises preferably a polymer insoluble in aqueous solutions with pH below 5.5, and at least one further pharmaceutically acceptable excipient selected from plasticisers such as polyethylene glycol, triethylcitrate, triacetine, propylenglycol which are preferably capable of decreasing the glass transition point of the methacrylic copolymer in concentration range from about 7 to 50% in relation to the weight of polymer unsoluble in pH below 5.5, to below 100°C, more preferably below 75°C and most preferably below 50°C.

In the gastro-resistant coating methacrylic acid copolymers are present in an amount of at least 5%, more preferably 10% to 50% and most preferably from 13% to 35% by the total weight of enteric coated pellets (pharmaceutical composition of the invention).

The gastro-resistant coating may further contain pharmaceutically acceptable plasticizers to obtain the desired mechanical properties, such as flexibility and hardness. The amount of plasticizer is preferably optimized for each gastro-resistant coating formula, in relation to the selected gastro-resistant coating polymer(s), selected plasticizer(s) and the applied amount of said polymer(s), in such a way that the mechanical properties, i.e. flexibility and hardness of the gastro-resistant coating(s) are adjusted so that the acid resistance of the pellets covered with gastro-resistant coating(s) does not decrease significantly during a compression of the pellets into tablets. The amount of plasticizer is usually about 7-50 % by weight of the gastro-resistant coating polymer(s). Additives such as dispersants, colorants, pigments, polymers, anti-tacking agent and antifoaming agents may also be included into the gastro-resistant coating(s). Other compounds may be added to increase film thickness and to decrease diffusion of acidic gastric juices into the acid susceptible material.

To protect an acidic susceptible substance the gastro-resistant coating in the present invention can have a thickness in the range from about 15 to about 120 µm, preferably from about 25 to about 100 µm. The maximum thickness of the applied gastro-resistant coating is normally only limited by processing conditions and dissolution of drug from pellets.

The aim of the gastro-resistant coating is to protect the ingredients present in the pharmaceutical composition according to the present invention from acidic environment. After entering into the media of suitable pH value the gastro-resistant barrier preferably dissolves and releases the components of the layers below. The functionality of the gastro-resistant coating can be tested according to the following procedure:

Gastro-resistant pharmaceutical composition in the context of this invention means that the dosage formulation exhibits a dissolution profile such that after 20 minutes 40 to 70 % of the active ingredient originally contained in a particle type is released, and after 45 minutes more than 80% of the active ingredient contained in a particle type is released after dissolving the dosage formulation in 1000 ml USP buffer pH 6.8 at 37° C using Apparatus 1 (Ph. Eur and USP baskets) at 100 rpm after firstly exposing the dosage formulation for 2 hours to USP Simulated Gastric Fluid pH 2.0 at 37 °C (all references herein to "USP" are meant to refer to US Pharmacopoeia Edition 23). Moreover gastro-resistant pharmaceutical composition in the context of this invention means that the dosage formulation exhibits good gastro-resistance. If gastro-resistant pharmaceutical composition according to the present invention is exposed to 2 hour treatment in 500 ml USP Simulated Gastric Fluid pH 2.0 at 37 °C in Apparatus 1 (Ph. Eur and USP baskets) at 100 rpm, determined assay as not less than 90 % of the active ingredient can be recovered.

The pharmaceutical composition according to the present invention can be optionally further coated with an over-coating layer based on hydrophilic polymer selected from the group consisting of, but not limited to, low viscosity hypromellose USP XXII types 2910 and 2208 (viscosity of 2% water solution at room temperature is below 20 cps), hyprollose, methylcellulose, polyvinyl alcohol, aminoalkyl methacrylate copolymers such as for example sold under trade name Eudragit E PO in order to obtain lower water intake of coated dosage form during storage and to improve the physical appearance of the surface of finished dosage form. Over-coating can include beside the polymer also antitacking agents such as for example talc, pigments, soluble colours. The preferred requirement for the over-coating is that it has to dissolve in artificial gastric juice at body temperature in less than about 5 minutes, preferably in less than about 4 minutes, most preferably in less than about 2 minutes.

The over-coating layer in the pharmaceutical composition according to the present invention can have a thickness of from about 1 to about 20 µm, preferably from about 2 to about 15 µm and most preferably from about 5 to about 10 µm.

The pharmaceutical composition according to the present invention can further comprise at least one pharmaceutically acceptable excipient selected from the group consisting of, but not limited to, binders, fillers, disintegrants, surfactants, glidants and antitacking agent. Said pharmaceutically acceptable excipient can be present in any part of the pharmaceutical composition according to the present invention.

Binders present in the pharmaceutical composition according to the present invention can be selected from the group consisting of, but not limited to, water soluble polymers such as for example, but not limited to, hypromellose USP XXII types 2910 and 2208 having viscosity of 2% solution in water at room temperature below 20cps, hydroxypropyl cellulose, povidone, copovidone, polyvinyl acetate, carboxymethylcellulose sodium, copolymer of polyvinyl alcohol and polyethylene oxide salt under trade name Kollicoat, methylcellulose, ethylcellulose, hydroxyethylcellulose, pharmaceutically acceptable substances having a melting point below about 90°C, preferably below about 70°C and most preferably below about 55°C, such as for example, but not limited to, polyethylene glycol, glycerol monostearate, alkyl macrogolglycerides such as those for example sold under trade name of Gelucire^{®} from Gatefose or poloxamers or water insoluble binders such as for example microcrystalline cellulose and any mixtures thereof.

Fillers present in the pharmaceutical composition according to the present invention can be selected from the group consisting of, but not limited to, water soluble pharmaceutically acceptable substances such as for example mono and disaccharides as for example sucrose, lactose, sugar alcohols as for example mannitol, xylitol, maltitol, fillers generally used in the pharmaceutical compositions such as for example microcrystalline cellulose, starch, dextrans and any mixtures thereof.

Disintegrants present in the pharmaceutical composition according to the present invention can be selected from the group consisting of, but not limited to, low substituted hydroxypropyl cellulose, crospovidone, crosscarmelose sodium, starch derivatives and any mixtures thereof. Said disintegrants can be incorporated into the pharmaceutical composition with dry procedures such as for example, but not limited to, direct pelletization or powder layering, which do not change the functionality of the disintegrators during manufacturing procedure.

Surfactants present in the pharmaceutical composition according to the present invention are used to [b3] improve the dissolution and improve the yield of coating by decreasing surface tension of the coating liquid. Nonionic surfactants selected from the group consisting of, but not limited to, sorbitane derivatives as for example polysorbates, poloxameres, sugar esters or ionic surfactants as for example metal alkyl sulphates as for example sodium lauryl sulphate or any mixture thereof can be used.

Glidants present in the pharmaceutical composition according to the present invention such as for example colloidal silica sold under trade name of Aerosil can be used in order to improve powder flowability in powder layering.

Antitacking agents present in the pharmaceutical composition according to the present invention such as for example talc or magnesium stearate can be used in order to decrease the stickiness of the pellets and their resulting agglomeration during the manufacturing process.

In the pharmaceutical composition of the present invention one or more of said pharmaceutically acceptable excipients is present in an amount from 5% to 95%, more preferably from 20% to 90% and most preferably from 50% to 85% by weight of the pharmaceutical composition of the invention.

The second embodiment of the present invention relates to the preparation of the pharmaceutical composition which comprises an active core comprising duloxetine or its pharmaceutically acceptable derivatives, a separating layer comprising a water soluble alkaline substance, a gastro-resistant coating comprising gastro-resistant polymer selected from methacrylic acid copolymers and optionally an over-coating layer.

Pharmaceutical composition according to the present invention can be obtained by multiple coating steps.

The active core can be prepared from powders comprising duloxetine or its pharmaceutically acceptable derivatives and at least one pharmaceutically acceptable excipient by direct pelletization using state of the art pharmaceutical equipment for pelletization such as for example, but not limited to, extruders and spheronizators, rotor fluid bed equipment, high shear mixers designed for spheronization of obtained agglomerates. The products of said process are matrix pellets, wherein duloxetine or its pharmaceutically acceptable derivatives is preferably homogeneously distributed in the pellet body.

The active core in the form of tablet can be produced by state of the art processes such as for example; but not limited to, direct compression of duloxetine or its pharmaceutically acceptable salts in admixture with at least one excipient, compression of a pregranulated mixture of duloxetine or its pharmaceutically acceptable derivatives and one or more pharmaceutically acceptable excipients selected from the group consisting of, but not limited to, binders, fillers, disintegrants, lubricants, and glidants. Granulation step can be performed either by a wet procedure, where solvents such as for example water or water miscible organic solvents are used or by a dry procedure such as for example slugging or compaction.

The active core can be formed by applying a layer containing duloxetine or its pharmaceutically acceptable derivatives to an inert bead. A convenient manner of coating the beads with duloxetine or its pharmaceutically acceptable derivatives can be the powder layering process which is performed using state of the art functional equipment such as for example, but not limited to, rotor tangential fluid bed systems, nonperforated pan coaters, rotating plate equipment or bottom spray fluid bed equipment, where rotor tangential fluid bed systems such as those produced by Glatt GmbH (Binzen Germany) and rotating plate equipment such as Freud CF-Granulator, produced by Vector Corp. (Marion, USA) are preferred. The inert beads are moistened with a solution of binder, and then the active substance together with other excipients is added as a powder and the layered pellets are dried in the same equipment as the coating is performed or other specialized equipment for drying, such as a drying chamber with or without vacuum.

Alternatively, the layer with duloxetine or its pharmaceutically acceptable derivatives can be formed in the suspension layering process, performed in state of the art fluid bed equipment such as for example, but not limited to, bottom spray systems, such as for example obtainable by Glatt GmbH (Binzen Germany), Niro Pharma systems (Bubendorf, Swiss), Hüttlin GmbH (Steinen, Germany), top or tangential spray systems, or classical nonperforated pan coaters. Duloxetine or its pharmaceutically acceptable derivatives is dispersed together with at least one acceptable excipient in a suitable liquid where duloxetine or its pharmaceutically acceptable derivatives shows only partial solubility such as water, organic solvents with boiling point below 80°C or mixtures thereof. The suspension layering process is performed by spraying the pharmaceutically acceptable salt suspension onto the inert cores in a fluid bed coater granulator.

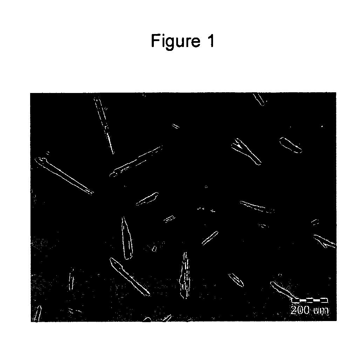

Coating with duloxetine or its pharmaceutically acceptable derivatives particles in a large needle-like form as depicted in figure 1 may be difficult.

In order to assure optimal coating procedure (maximal yield of coating) during the layering of duloxetine or its pharmaceutically acceptable salts onto the neutral cores, it is advisable to reduce the particle size of duloxetine or its pharmaceutically acceptable salts in order to achieve approximately 90% by volume of the particles below about 35 µm with an average diameter of less than about 40 µm, preferably less than about 30 µm and most preferably less than about 20 µm having specific area of at least about 0.5m²/g, preferably at least about 1.0m²/g (as measured by gas sorption system Tristar 3000 based on nitrogen adsorption, using 6 point Brunauer, Emmett and Teller (BET) method with prior degassing of the sample at room temperature).

The size of the particles is determined by laser light scattering method using for example a Malvern Mastersizer 2000 Apparatus with vegetable oil as the dilution medium. Particle sizes are determined by measuring the angular distribution of laser light scattered by a homogeneous suspension of particles.

Solution layering process can be performed in the same equipment as described for suspension layering. The main difference is that a liquid is selected from solvents in which duloxetine or its pharmaceutically acceptable salts are soluble at least in a ratio 20 w/v %.

The pharmaceutical composition of the present invention comprises one or more separating layers wherein at least one of said separating layers contains a water soluble alkaline compound.

The separating layer can be applied to the active core by a coating or layering process in a suitable equipment such as for example, but not limited to, a coating pan, coating granulator or in fluid bed apparatus using water and/or organic solvents for the coating process. As an alternative the separating layer can be applied to the active core by using the powder coating technique.

One or more gastro-resistant coatings are applied onto the active core covered with at least one separating layer comprising the water soluble alkaline substance by using a suitable coating technique. The gastro-resistant coating material may be dispersed or dissolved in either water or in suitable organic solvents.

The separating layer, gastro-resistant coating and over-coating layer can be applied to the pellets by coating or layering procedures in suitable equipment, such as a coating pan, a coating granulator or a fluidized bed apparatus using water and/or organic solvents for the coating process.

The third embodiment of the present invention relates to the use of a water soluble alkaline substance in a separating layer in order to avoid interaction between duloxetine or its pharmaceutically acceptable derivatives in the active core and gastro-resistant polymer, particularly methacrylic acid copolymers, in the gastro-resistant coating that could occur during in-vivo dissolution of duloxetine or its pharmaceutically acceptable derivatives.

Due to its acidic nature and steric characteristics, gastro-resistant polymers from the group of methacrylicates are likely to react with some alkaline components present in the pharmaceutical composition. It was noticed that the dissolution rate is significantly increased when introducing an alkaline component in the separating layer. Moreover, the position of said compound into the separating layer prevents the contact between active ingredient and methacrylate, and enables the sequence of dissolution: after the gastro-resistant polymer is dissolved, the separating layer is subject to hydration and diffusion, prior to dissolution of active layer.

The fourth embodiment of the present invention relates to the process for the preparation of duloxetine hydrochloride with appropriate chemical and enantiomeric purity and with particle size appropriate for direct use in the pharmaceutical composition according to the present invention.

It is advantageous to prepare duloxetine or its pharmaceutically acceptable derivatives as for example duloxetine hydrochloride with particles size appropriate for direct use in the pharmaceutical composition. When precipitating duloxetine hydrochloride by addition of a solution of duloxetine hydrochloride in a solvent to antisolvent, the temperature of the antisolvent plays a significant role in controlling the agglomeration of duloxetine hydrochloride precipitate. Appropriate solvents can be selected from the group consisting of, but not limited to, C1-C4 alcohols, acetonitrile, acetic acid, preferably methanol, ethanol, acetic acid, and appropriate anti-solvent that can be selected from group consisting of, but not limited to, C3-C8 esters, C3-C8 ketones, C2-C8 ethers, C5-C8 straight, branched or cyclic alkanes, preferably ethyl acetate, ethyl methyl ketone or MTBE. Additionally, solvents mentioned above can be used as antisolvents in the process disclosed at temperatures below 15°C, preferably below 0°C. During the precipitation antisolvent can be maintained at temperatures between about -10 and about 50°C, preferably about 10 to about 30°C.

In this embodiment, duloxetine hydrochloride particles size appropriate for direct use in pharmaceutical formulation can be obtained by controlling temperature of nucleation of duloxetine hydrochloride solution during the crystallisation process/cooling in a narrow range. We suprisingly found out, that with control of nucleation temperature within a very narrow range of 5°C it is possible to control primary particle size between 20 µm and 150 µm. Further nucleation can be controlled by means of starting concentration of duloxetine in crystallization process, stirring, rate of cooling, ultrasound or by seeding.

The invention is illustrated by the following examples. The examples do not intend to limit the scope of this invention as defined in the claims below.

### Examples:

Example 1 (Comparative): Pharmaceutical composition according to WO 2007093439:

| **Ingredients** | **Composition** |
|---|---|
| **Active core** | |
| Sucrose-starch nonpareils | 114,00 mg |
| Duloxetine hydrochloride | 67,35 mg |
| Hydroxypropyl methylcellulose | 8,70 mg |

| **Separating layer** | |
|---|---|
| Hydroxypropyl methylcellulose | 10,21 mg |
| Talc | 20,00 mg |
| Sucrose | 10,83 mg |

| **Gastro-resistant coating** | |
|---|---|
| Methacrylic acid ethyl acrylate copolymer | 64,41 mg |
| Macrogol 6000 | 19,32 mg |
| Talc | 6,44 mg |
| TiO₂ | 6,44 mg |
| Polysorbate 80 | 2,90 mg |

| **Over-coating layer** | |
|---|---|
| Hydroxypropyl methylcellulose | 6,75 mg |
| Titanium dioxide | 10,00 mg |
| **Total** | **347,35 mg** |

The duloxetine layering was performed in a fluid bed processor using bottom spray technique (Wurster column) at a batch size of 1.5 kg. The duloxetine was suspended in the hydroxypropyl methylcellulose water solution. The suspension was slowly sprayed onto the agitating batch of beads. The prepared core material was dried and covered with separating layer in a Wurster column with a water solution of hydroxypropyl methylcellulose containing talc and sucrose.

The enteric coating suspension was prepared using methacrylic acid ethyl acrylate copolymer (Eudragit L 30 D-55), Macrogol 6000, talc, titanium dioxide and Polysorbate 80. The suspension was sprayed onto the pellets covered with separating layer in a fluid bed apparatus. The spray operation was stopped when the specified amount of bulk liquid had been sprayed, and then drying was carried in the Wurster column.

The final coating was applied in the Wurster column by spraying the dispersion of hydroxypropyl methylcellulose and titanium dioxide on the enteric-coated pellets.

Drying in the same apparatus followed the procedure. The pellets were filled into #1 gelatine capsules.

### Example 2:

### Pharmaceutical composition:

| **Ingredients** | **Composition** |
|---|---|
| **Active Core** | |
| Sucrose-starch nonpareils | 114,00 mg |
| Duloxetine hydrochloride | 67,35 mg |
| Hydroxypropyl methylcellulose | 8,70 mg |

| **Separating layer** | |
|---|---|
| Hydroxypropyl methylcellulose | 10,21 mg |
| Talc | 20,00 mg |
| Sucrose | 10,83 mg |
| Disodium hydrogen phosphate dihydrate | 5,00 mg |

| **Gastro-resistant coating** | |
|---|---|
| Methacrylic acid ethyl acrylate copolymer | 64,41 mg |
| Macrogol 6000 | 19,32 mg |
| Talc | 6,44 mg |
| TiO₂ | 6,44 mg |
| Polysorbate 80 | 2,90 mg |

| **Over-coating layer** | |
|---|---|
| Hydroxypropyl methylcellulose | 6,75 mg |
| Titanium dioxide | 10,00 mg |
| **Total** | **352,35 mg** |

The duloxetine layering was performed in a fluid bed processor using bottom spray technique (Wurster column) at a batch size of 1.5 kg. The duloxetine was suspended in the hydroxypropyl methylcellulose water solution. The suspension was slowly sprayed onto the agitating batch of beads. The prepared core material was dried and covered with separating layer in a Wurster column with a water solution of hydroxypropyl methylcellulose containing talc, sucrose and dibasic sodium phosphate.

The gastro-resistant coating suspension was prepared using methacrylic acid ethyl acrylate copolymer (Eudragit L 30 D-55), Macrogol 6000, talc, titanium dioxide and Polysorbate 80. The suspension was sprayed onto the pellets covered with separating layer in a fluid bed apparatus. The spray operation was stopped when the specified amount of bulk liquid had been sprayed, and then drying was carried in the Wurster column.

The final coating was applied in the Wurster column by spraying the dispersion of hydroxypropyl methylcellulose and titanium dioxide on the enteric-coated pellets. Drying in the same apparatus followed the procedure. The pellets were filled into #1 gelatine capsules.

In vitro dissolution studies of duloxetine capsules of Examples 1 and 2 were carried out in 1000 ml USP buffer pH 6.8 at 37 °C in Apparatus 1 (Ph Eur and USP baskets) at 100 rpm after firstly dissolving the pharmaceutical composition for 2 h in USP simulated gastric fluid pH 2.0 at 37 °C.

The dissolution profiles in the following table reveal the difference between both formulations. The advantage of the pharmaceutical composition containing water soluble alkaline substance according to the present invention is confirmed with increased dissolution profile.

**Table 1: In-vitro dissolution results of duloxetine capsules 60 mg from Comparative Example 1 and Example 2 in pH 6.8**

| **Time (min)** | **Av. % drug release** | |
|---|---|---|
| | **Duloxetine capsules** | **Duloxetine capsules** |
| | **Example 1** | **Example 2** |
| 0 | 0 | 0 |
| 10 | 8 | 16 |
| 15 | 24 | 44 |
| 20 | 36 | 59 |
| 30 | 51 | 70 |
| 45 | 61 | 81 |
| 60 | 67 | 84 |

### Example 3:

### Pharmaceutical composition:

| **Ingredients** | **Composition** |
|---|---|
| **Active core** | |
| Sucrose-starch nonpareils | 100,00 mg |
| Duloxetine hydrochloride | 67,35 mg |
| Hydroxypropyl methylcellulose | 8,70 mg |
| Sucrose | 10,00 mg |

| **Separating layer** | |
|---|---|
| Hydroxypropyl methylcellulose | 14,00 mg |
| Talc | 25,00 mg |
| Disodium hydrogen phosphate dihydrate | 5,00 mg |

| **Gastro-resistant coating** | |
|---|---|
| Methacrylic acid ethyl acrylate copolymer | 64,41 mg |
| Macrogol | 19,32 mg |
| Talc | 6,44 mg |
| TiO₂ | 6,44 mg |
| Polysorbate 80 | 2,90 mg |
| **Total** | **329,56 mg** |

The duloxetine layering was performed in a fluid bed processor using bottom spray technique (Wurster column) at a batch size of 1.5 kg. The duloxetine was suspended in the water solution of hydroxypropyl methylcellulose and sucrose. The suspension was slowly sprayed onto the agitating batch of beads. The prepared core material was dried and covered with separating layer in a Wurster column with a water solution of hydroxypropyl methylcellulose and disodium hydrogen phosphate dihydrate containing talc.

The gastro-resistant coating suspension was prepared using methacrylic acid ethyl acrylate copolymer (Eudragit L 30 D-55), Macrogol 6000, talc, titanium dioxide and Polysorbate 80. The suspension was sprayed onto the pellets covered with separating layer in a fluid bed apparatus. The spray operation was stopped when the specified amount of bulk liquid had been sprayed, and then drying was carried in the Wurster column. The pellets were filled into #1 gelatine capsules.

Duloxetine hydrochloride used in the examples above could be prepared by any method known from the state of the art. Duloxetine hydrochloride could be obtained by the following method as well:

### Example 4: Synthesis of (S)-N,N-dimethyl-3-(1-naphthalenyloxy)-3-(2-thienyl)propanamine oxalate

In 50 ml of dimethylacetamide 0.5 g of sodium hydride (suspension in mineral oil) is suspended. Suspension is stirred and in few portions 2 g of *(s)*-3-(dimethylamino)-1-(thienyl)-propan-1-ol is added. After the addition is completed the suspension is warmed up to 70°C. 1.27 ml of 1-fluoronaphthalene is added and the reaction mixture is heated to 110°C. At this temperature the solution is stirred for another three hours. Then the reaction solution is dissolved with 300 ml of water and extracted twice with 100 ml ether. Combined ether phases are rinsed with water, dried with sodium sulphate end evaporated to dryness. Obtained oil is dissolved in 50 ml ethyl acetate and 0.9 g of oxalic acid is added. The suspension is further stirred for one hour, filtered off the product and washed with ethyl acetate. The product is dried to obtain 3.5 g (80 %) of (S)-N,N-dimethyl-3-(1-naphthalenyloxy)-3-(2-thienyl)propanamine oxalate.

### Example 5: Synthesis of (S)-N-methyl-3-(1-naphthalenyloxy)-3-(2-thienyl)propanamine hydrochloride

To a stirred mixture of of (S)-N,N-dimethyl-3-(1-naphthalenyloxy)-3-(2-thienyl)propanamine oxalate (3 g, 7.48 mmol) in 50 ml toluene, 50 ml of water and 5 ml of 30 % ammonium hydroxide is added. The suspension is stirred for another two hours, the layers are separated. The organic phase is washed with water, dried with sodium sulphate and filtered. The filtrate is concentrate to dryness and another 60 ml of toluene is added. The reaction mixture is heated to reflux temperature and 1.03 ml of phenylchloroformate is added dropwise. The refluxing solution was refluxed for another two hours and cooled to room temperature. The solution is washed with 2 M NaOH, water, 1 M HCl and brine and the organic phase is evaporated to dryness. The residue is dissolved in 80 ml of propylene glycol, 15 ml of 5 M NaOH is added and heated at 110°C for another 1.5 hours. The reaction mixture is diluted with water (200 ml), extracted with ether (100 ml). The organic phase is washed with brine, dried with sodium sulphate, filtered and the ether phase is evaporated to dryness. The obtained oil is dissolved in 50 ml of ethyl acetate and dry hydrogen chloride is introduced to saturation. The suspension is stirred for another hour at 0°C, filtered and washed with cold ethyl acetate. The product is vacuum dried to constant weight to give 1.8 g (75 %) dry duloxetine hydrochloride.

### Example 6: Crystallization of duloxetine hydrochloride in 2-propanol

5 g of duloxetine hydrochloride is dissolved in 40 ml 2-propanol at reflux temperature. The solution is filtered and during intensive stirring the temperature is lowered to about 0°C. At this temperature the suspension is stirred for another two hours and filtered to yield the product. The wet product on filter is rinsed with 10 ml of acetone. The product is dried and obtained as 4.3 g of pure duloxetine hydrochloride (86 %) with chromatographic purity more than 99.95 %.

### Example 7: Precipitation of duloxetine hydrochloride from methanol/ethylacetate solvent system

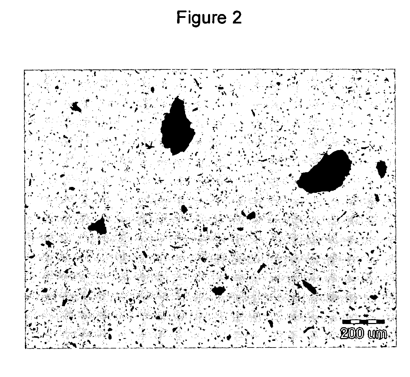

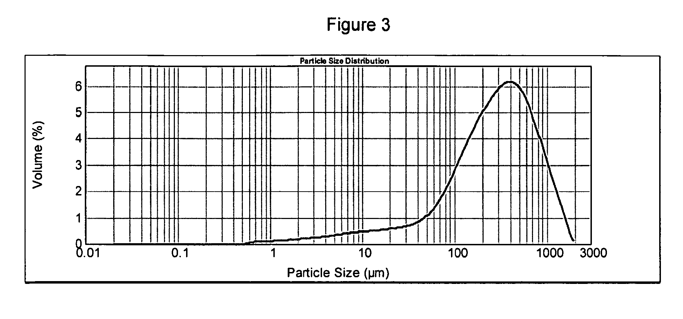

5 g of duloxetine hydrochloride was suspended in 7.5 mL of methanol and stirred at 50 °C until a clear solution was obtained. The clear solution was added to 65 mL of ethylacetate at 0 °C. Suspension was stirred with an overhead stirrer for 3 hours. The product was collected and washed with 10 mL of ethylacetate. Agglomerates of relatively small particles were obtained as shown in figure 2, parictle size distribution in figure 3. The yield was 86%.

### Example 8: Precipitation of duloxetine hydrochloride from methanol/ethylacetate solvent system

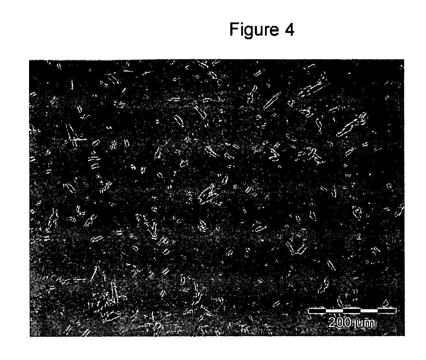

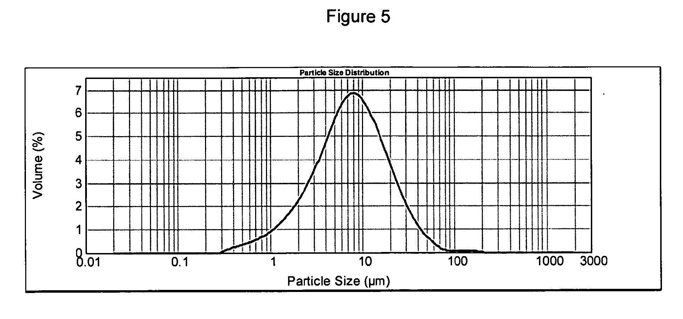

5 g of duloxetine hydrochloride was suspended in 7.5 mL of methanol and stirred at 50 °C until a clear solution was obtained. The clear solution was added to 65 mL of ethylacetate at 15 °C. The suspension was stirred with an overhead stirrer for 3 hours. The product was collected and washed with 10 mL of ethylacetate. Particles appropriate for direct use in pharmaceutical processes were obtained as shown in figure 4, particle size distribution in figure 5. The yield was 84%.

### Example 9: Purification of duloxetine hydrochloride from 2-propanol/acetone solvent system

### A)

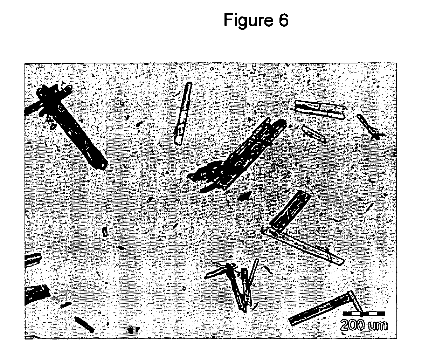

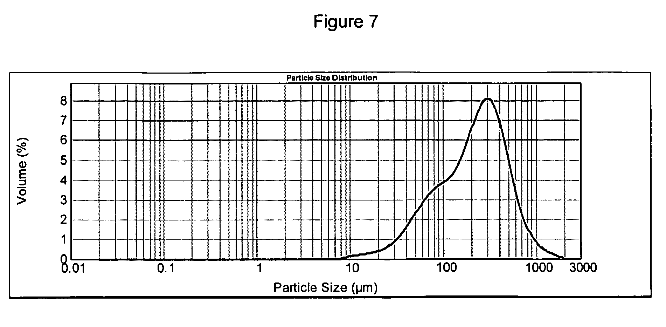

10g of duloxetine hydrochloride was suspended in 2-propanol. Suspension is heated until clear solution is obtained. The clear solution was cooled to 55 °C in one hour and allowed to crystallize for one hour at the same temperature. Nucleation started at 67°C. Then the suspension was gradually cooled to 20 °C in three hours. The product was collected by filtration and washed with 10 mL of acetone. Large needle like crystals were obtained as showed in figure 6, particle size distribution in figure 7. The yield was 91 %.

### B)

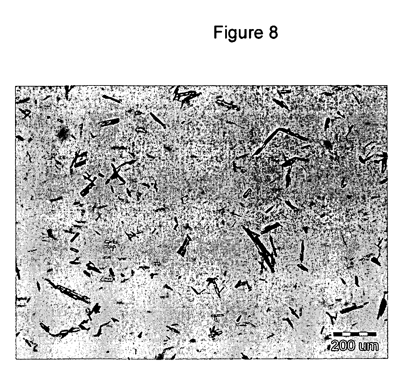

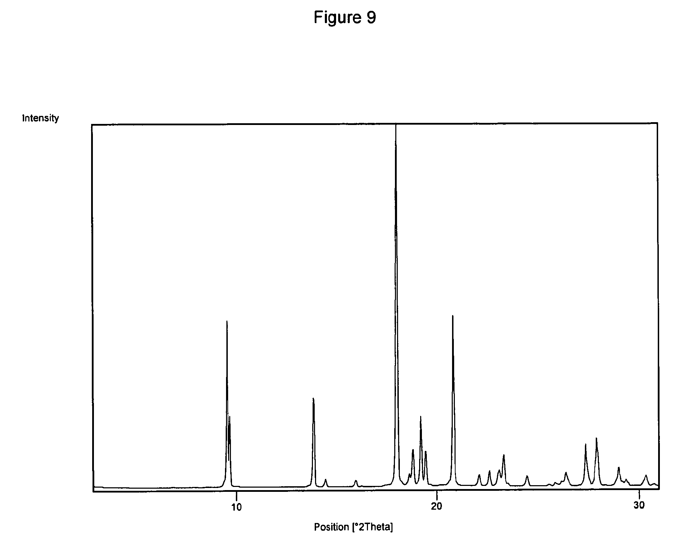

Duloxetine hydrochloride was prepared by the same way as described above with the exception that the cooling rate in the first step was faster and stirring of the solution was more intense. Nucleation started at 62°C and smaller needle like crystals (average size less than 40µm) were obtained. Particles of duloxetinehydrochloride are presented in Figure 8 and X-ray powder diffraction pattern of duloxetinehydrochloride in figure 9.

### Example 10: Purification of duloxetine hydrochloride in mixture acetic acid/acetone

2 g of duloxetine hydrochloride is suspended in 3 ml of acetic acid. The suspension is stirred and the temperature is raised up to 40°C. After all duloxetine hydrochloride is dissolved, 25 ml of acetone is added dropwise. The clear solution is further chilled and at about room temperature the crystallization began. The suspension was chilled to about 0°C and at this temperature the suspension is stirred for another two hours. The product is filtered off, washed with acetone and dried. 1.3 g of pure duloxetine hydrochloride was obtained.

### Example 11: Precipitation of duloxetine hydrochloride in mixture methylethylketone /methanol

5 g of duloxetine hydrochloride is dissolved in 7.5 ml of methanol at about 50°C. In another reaction flask equipped with a mechanical stirrer 100 ml of methylethylketone is prepared. The hot methanol solution of duloxetine hydrochloride was introduced in methylethylketone during intense stirring. The crystalissation began immediatelly. The obtained solution was cooled to about 0°C, stirred for another hour and filtered off the product. After drying, 3.3 g of pure duloxetine hydrochloride was obtained.

## Claims

1. Pharmaceutical composition, comprising at least
a. an active core comprising duloxetine or its pharmaceutically acceptable derivatives,
b. a separating layer comprising one or more water soluble alkaline substances, and
c. a gastro-resistant coating comprising one or more gastro-resistant polymers, wherein at least one is preferably selected from methacrylic acid copolymers,
in this order.

2. Pharmaceutical composition according to claim 1, further comprising an over-coating layer outside the gastro-resistant coating.

3. Pharmaceutical composition according to claim 1 or 2, wherein the water soluble alkaline substance, when dissolved in concentration of 10 w/vol% in purified water, gives the pH of solution at 25°C in the range 7 to 13.

4. Pharmaceutical composition according to claim 3, wherein the water soluble alkaline substance is selected from sodium or potassium salt of phosphoric, carbonic, or citric acid, or is a basic amino acid, and esters or salts thereof, or an amino carbohydrate derivative such as meglumine.

5. Pharmaceutical composition according to any one of claims 1 to 4, wherein the water soluble alkaline compound in the separating layer is present in an amount from 0.1% to 10%, more preferably from 0.5% to 5% by weight of the pharmaceutical composition of the invention.

6. Pharmaceutical composition according to any one of claims 1 to 5, wherein the gastro-resistant polymer is methacrylic acid ethyl acrylate copolymer.

7. Process for the preparation of the pharmaceutical composition according to any one of claims 1 to 6, which comprises the steps of
a. forming an active core comprising duloxetine or its pharmaceutically acceptable derivatives;
b. coating the active core with at least one separating layer comprising one or more water soluble alkaline substances, and
c. applying at least one gastro-resistant coating comprising one or more gastro-resistant polymers, wherein at least one is preferably selected from methacrylic acid copolymers.

8. Use of a water soluble alkaline substance in a separating layer of a pharmaceutical composition comprising duloxetine or its pharmaceutically acceptable derivatives in the active core and comprising a gastro-resistant coating containing a gastro-resistant polymer as an outer layer, to improve dissolution of duloxetine or its pharmaceutically acceptable derivatives.

9. Use according to claim 8, wherein the gastro-resistant polymer is a methacrylic acid copolymer.

10. Process for the preparation of duloxetine hydrochloride, comprising the steps of
a. forming a solution of duloxetine hydrochloride in a solvent; and
b. precipitating duloxetine hydrochloride by mixing said solution of duloxetine hydrochloride with an antisolvent,
wherein the temperature of the antisolvent is within the range of from 0 to 50°C.

11. Process according to claim 10, wherein the temperature of the antisolvent is within the range of from 10 to 30°C.

12. Process according to claim 10 or 11, wherein the temperature of the antisolvent is controlled to be constant within a range of 5°C.
